# EUROPÄISCHE PATENTSCHRIFT

(11) **EP 0 850 937 B1**
(45) Veröffentlichungstag und Bekanntmachung des Hinweises auf die Patenterteilung: **12.03.2003**
(21) Anmeldenummer: 97122262.5
(22) Anmeldetag: 17.12.1997
(51) Int. Cl.: C07D 311/72

(54) **Verfahren zur Herstellung von alpha-Tocopherol oder alpha-Tocopherylacetat durch Umsetzen von Trimethylhydrochinon und Phytol oder Isophytol unter Rückführung des Zinkhalogenid-Kondensationskatalysators**
Process for the preparation of alpha-tocopherol or alpha-tocopheryl acetate by reacting trimethylhydroquinone and phytol or isophytol and recycling the zinc halogenide condensation catalyst
Procédé pour la préparation d'alpha-tocophérol ou d'acétate d'alpha-tocophérol par réaction de la triméthylhydroquinone avec le phytol ou l'isophytol comprenant le recyclage d'halogénure de zinc comme catalyseur de condensation

(30) Priorität: 23.12.1996 DE 19654038; 01.08.1997 DE 19733503
(43) Veröffentlichungstag der Anmeldung: 01.07.1998
(73) Patentinhaber: BASF AKTIENGESELLSCHAFT, 67056 Ludwigshafen (DE)
(72) Erfinder: Baldenius, Kai-Uwe, Dr., 67227 Frankenthal (DE); Kaiser, Wulf, Dr., 67098 Bad Dürkheim (DE); Bockstiegel, Bernhard, Dr., 67354 Römerberg (DE); Laas, Harald, Dr., 67133 Maxdorf (DE); Schulz, Bernhard, Dr., 68723 Schwetzingen (DE); Schmitt, Peter, 67069 Ludwigshafen (DE); Glietenberg, Helmut, Dr., 67071 Ludwigshafen (DE)

(56) Entgegenhaltungen:
- EP-A- 0 087 576
- EP-A- 0 100 471
- EP-A- 0 694 541
- DE-A- 2 606 830
- DE-A- 2 743 920
- US-A- 2 411 969
- US-A- 3 708 505
- M. MATSUI ET AL.: BULL. CHEM. SOC. JPN., Bd. 68, Nr. 12, 1995, Seiten 3569-71, XP002060104
- M. MATSUI ET AL.: BULL. CHEM. SOC. JPN., Bd. 69, Nr. 1, 1996, Seiten 137-9, XP002060105

## Beschreibung

Die Erfindung betrifft ein verbessertes Verfahren zur Herstellung von α-Tocopherol oder α-Tocopherylacetat durch Umsetzen von 2,3,5-Trimethyl-hydrochinon (TMH) mit einem Phytol, insbesondere mit Isophytol (IP) in Gegenwart von einem Zinkhalogenid, einem Protonendonator und gegebenenfalls in Gegenwart von einem Amin in einem Lösungsmittel bei erhöhter Temperatur und gegebenenfalls anschließende Veresterung mit Acetanhydrid, unter Rückführung des Zinkahlogenid-Kondensationskatalysators. Unter einen Zinkhalogenid-Kondensationskatalysator werden hierbei im wesentlichen die preiswert erhältlichen Halogenide Zinkchlorid und Zinkbromid verstanden, aber auch Mischungen derselben sowie Mischungen derselben mit basischen Chloriden und Bromiden des Zinks, d.h. Oxy- und Hydroxyhalogenide.

In den vergangenen Jahren hat α-Tocopherol (Vitamin E) als Antioxidans sowie auf dem Gebiet der menschlichen und tierischen Ernährung in steigendem Maße Bedeutung erlangt. Zu seiner Synthese sind daher zahlreiche Verfahren entwickelt worden. Eine Übersichtsarbeit von Siebrell und Harris hierüber findet sich in "The Vitamins", Vol V, Seiten 168 f (1972).

In industriellem Maßstab hat es sich bewährt, Vitamin E durch Kondensation von TMH mit IP in einem Lösungsmittel in Gegenwart von Lewissäuren, insbesondere von Zinkchlorid, zusammen mit einem Protonendonator, insbesondere von Chlorwasserstoffgas bei erhöhter Temperatur umzusetzen (vgl. US 24 11 969 der Firma Hoffmann-La Roche, US 4 239 691 der Firma Eastman Kodak, US 3 708 505 der Firma Diammond Shamrock und DE 3 203 487 der Firma BASF).

Eine breite Palette von Lösungsmitteln kann für diese Umsetzung eingesetzt werden, z.B. Ethylacetat, Essigsäure, aliphatische oder aromatische Kohlenwasserstoffe oder Chlorkohlenwasserstoffe.

Gemäß DE 26 06 830 und EP 100 471 erhält man bei dieser Umsetzung besonders gute Ausbeuten und besonders reines Vitamin E, wenn man die Umsetzung zusätzlich in Gegenwart von geringen Mengen eines Amins oder eines Ammoniumsalzes durchführt.

Nachteilig an diesen an sich recht guten Verfahren ist, daß durch die übliche Verwendung von größeren Mengen an ZnCl₂ große Abwasserprobleme auftreten. Ein weiterer großer Nachteil bei der Verwendung von Zinkchlorid für diese Umsetzung ist gemäß Bull. Chem. Soc. Jpn., 68 (1995), 3569-71, insbesondere 3569, linke Spalte, und Bull. Chem. Soc. Jpn. 69 (1996), 137-139, insbesondere 137, linke Spalte, daß Lewis-Säuren, wie Zinkchlorid, durch das bei der Umsetzung gebildete Wasser desaktiviert werden.

Versuche, das Zinkchlorid mit Wasser aus dem Reaktionsgemisch zu extrahieren und es in Form der erhaltenen 20 bis 60 gew.-%igen Lösung in die Umsetzung zurückzuführen, führten zu schlechten Ausbeuten und zu weniger reinem Vitamin E. Eine separate Aufarbeitung der 20- bis 60 gew.-%igen Zinkchlorid-Lösung zu trockenem Zinkchlorid-Pulver und Rückführung des Zinkchlorid-Pulvers in die Vitamin-E-Synthese ist aufgrund des hohen apparativen Aufwands für das Arbeiten mit einem Feststoff nicht ökonomisch durchführbar.

Wegen dieser Schwierigkeiten beim Arbeiten mit Zinkchlorid wurden in der neuesten Literatur für die Kondensation von TMH und IP in industriellem Maßstab andere saure Katalysatoren beschrieben. So wird beispielsweise in Bull. Chem. Soc. Jpn. 68 (1995), 3569 bis 3571, Scandium(III)trifluormethansulfonate und in Bull. Chem. Soc. Jpn. 69 (1996), 137-39, Montmorillonite mit ausgetauschten Metallionen als Katalysatoren empfohlen. In DE-OS 2 743 920 wird an Kieselgel/Aluminiumoxid adsorbiertes Zinkchlorid als Katalysator beschrieben.

Nachteilig an diesen Verfahren ist, daß Sc(III)trifluormethansulfonat sehr teuer und nicht in ausreichendem Maße verfügbar ist und die Montmorillonite bzw. die Zinkchlorid-Kieselgel/Aluminium-Katalysatoren für eine Wiederverwendung ein aufwendiges Feststoff-Handling erfordern.

Üblicherweise wird bei Verwendung von Zinkchlorid als Kondensationsmittel dieses als festes Pulver verwendet. In technischem Maßstab muß beim Einbringen eines Feststoffes in einen organische Lösungsmittel, wie Heptan, enthaltenden Reaktionskessel ein beträchtlicher technischer Aufwand getrieben werden um elektrische Aufladungen und die Bildung von explosionsfähigen Gemischen sowie die hierdurch auftretende Gefahr einer Explosion zu verhindern.

Es war daher die Aufgabe der Erfindung das an sich in der Technik bewährte Verfahren zur Herstellung von Vitamin E durch Kondensation von TMH mit IP in einem Lösungsmittel in Gegenwart von einem Zinkhalogenid, einem Protonendonator und ggf. in Gegenwart von einem Ammoniumsalz und/oder einem Amin so zu verbessern, daß man das aufwendige Feststoffhandling ohne Verschlechterung von Ausbeute und/oder Reinheit des α-Tocopherols bzw. α-Tocopherylacetats vermeiden kann. Es war weiterhin die Aufgabe, das Verfahren so zu verbessern, daß man das Zinkhalogenid und möglichst auch das Ammoniumsalz und/oder das Amin nach der Umsetzung auf einfache und ökonomische Weise zurückgewinnen und in die Umsetzung zurückführen kann.

Es wurde nun überraschenderweise gefunden, daß man bei dem Verfahren zur Herstellung von α-Tocopherol durch Umsetzen von TMH mit Phytol oder IP in Gegenwart von einem Zinkhalogenid-Kondensationskatalysator, ausgewählt aus der Gruppe Zinkchlorid oder Zinkbromid, Mischungen derselben sowie Mischungen derselben mit basischen Chloriden und Bromiden des Zinks und einem Protonendonator in einem Lösungsmittel auch dann reines α-Tocopherol in sehr guten Ausbeuten erhält, wenn man
A. die Umsetzung in einem mit Wasser nicht oder nur wenig mischbaren unpolaren Lösungsmittel durchführt und
B. das benötigte Zinkhalogenid in Form einer Mischung aus 1 bis 4 mol Wasser pro mol Zinkhalogenid, vorzugsweise 1 bis 3 mol Wasser pro mol Zinkhalogenid in die Umsetzung einbringt, was technisch einer etwa 65- bis 90-gew.-%igen, vorzugsweise einer etwa 70 bis etwa 90 gew.-%igen ggf. heißen wäßrigen Lösung, übersättigten Lösung oder pumpbaren Maische entspricht.

Es wurde weiterhin gefunden, daß diese etwa 65- bis 90 gew.-%igen Gemische aus Wasser und Zinkchlorid oder Zinkbromid bei Temperaturen von 20 bis 200°C, vorzugsweise 50 bis 200°C, durch entsprechend erwärmte Rohrleitungen transportiert und auf einfache Weise dosiert werden können.

Gegenstand der Erfindung ist dementsprechend ein Verfahren zur Herstellung von α-Tocopherol oder α-Tocopherylacetat durch Umsetzen von 2,3,5-Trimethyl-hydrochinon mit Phytol oder Isophytol in Gegenwart von einem Zinkhalogenid-Kondensationskatalysator, ausgewählt aus der Gruppe Zinkchlorid oder Zinkbromid, Mischungen derselben sowie Mischungen derselben mit basischen Chloriden und Bromiden des Zinks und einem Protonendonator in einem Lösungsmittel und ggf. anschließendes Verestern mit Acetanhydrid, das dadurch gekennzeichnet ist, daß man
A. die Umsetzung in einem mit Wasser nicht oder nur wenig mischbaren unpolaren Lösungsmittel durchführt und
B. das benötigte Zinkhalogenid in Form einer Mischung aus 1 bis 4 mol Wasser pro mol Zinkhalogenid, vorzugsweise 1 bis 3,5 mol Wasser pro mol Zinkhalogenid, insbesondere 1 bis 3 mol Wasser in die Umsetzung einbringt.

Ganz besondere Vorteile bringt die Möglichkeit, bei dem erfindungsgemäßen Verfahren das benötigte Zinkhalogenid in Form einer leicht handhabbaren Mischung aus 1 bis 4 mol Wasser pro mol Zinkhalogenid in die Umsetzung einbringen zu können für die Rückführung des Zinkhalogenid nach erfolgter Umsetzung.

Mit besonderem Vorteil gelingt das erfindungsgemäße Verfahren, wenn man als Zinkhalogenid eines der preiswert zugänglichen Halogenide Zinkchlorid oder Zinkbromid verwendet.

Gegenstand der Erfindung ist dementsprechend auch ein Verfahren zur Herstellung von α-Tocopherol oder α-Tocopherylacetat durch Umsetzen von TMH mit IP oder Phytol in Gegenwart von einem Zinkhalogenid-Kondensationskatalysator, ausgewählt aus der Gruppe Zinkchlorid oder Zinkbromid, Mischungen derselben sowie Mischungen derselben mit basischen Chloriden un Bromiden des Zinks und einem Protonendonator in einem Lösungsmittel und gegebenenfalls anschließende Veresterung mit Acetanhydrid, das dadurch gekennzeichnet ist, daß man
A. die Umsetzung in einem mit Wasser nicht oder nur wenig mischbaren unpolaren Lösungsmittel durchführt,
C. nach erfolgter Umsetzung das Zinkchlorid oder Zinkbromid abtrennt und/oder mit Wasser oder einem Gemisch aus Wasser und einem niedrig siedenden, mit Wasser mischbaren organischen Lösungsmittel aus der erhaltenen Tocopherol-Lösung extrahiert und
D. die erhaltene Zinkchlorid- bzw. Zinkbromid-Lösung ggf. nach Aufkonzentrieren in Form einer etwa 60 bis 90 gew.-%igen gegebenenfalls heißen Lösung oder pumpbaren Maische teilweise oder vollständig so in den Umsetzungsprozeß zurückführt, daß im Reaktionsgemisch nach Ergänzung des noch fehlenden Zinkchlorids bzw. Zinkbromids für die folgende Umsetzung 1 bis 4 mol Wasser, vorzugsweise nicht mehr als 3,5 mol Wasser, insbesondere nicht mehr als 3 mol Wasser, pro mol Zinkchlorid bzw. Zinkbromid vorliegen.

Das Rückführen der Zinkchlorid- oder Zinkbromid-Lösung nach dem Aufkonzentrieren in Form einer Lösung oder pumpbaren Maische anstelle von Zinkchlorid- oder Zinkbromid-Pulver ist möglich, wenn man im Verfahrensschritt D. die wäßrige Zinkchlorid- bzw. Zinkbromid-Lösung nach dem Aufkonzentrieren auf etwa 60 bis 90 Gew.-% Zinkchlorid bzw. Zinkbromid, vorzugsweise 70 bis 90 Gew.-% Zinkchlorid bzw. Zinkbromid, bei Temperaturen von 20 bis 200°C, vorzugsweise 50 bis etwa 200°C, aufbewahrt und/oder per entsprechend beheizter Rohrleitung in den Umsetzungsprozeß zurückführt.

Als nicht oder nur wenig mit Wasser mischbare unpolare Lösungsmittel kommen für das erfindungsgemäße Verfahren insbesondere Kohlenwasserstoffe, mit einem Siedepunkt oder Siedebereich von 60 bis 200°C, wie Hexan, Heptan, Cyclohexan, Octan, Nonan, Decan, Decalin, Toluol, Xylol oder Chlorbenzol oder aber Gemische aus zwei oder mehreren dieser Lösungsmittel, wie Petrolether, in Betracht. Mit besonderem Vorteil arbeitet man in Heptan. Prinzipiell kann aber auch Tocopherol selbst als unpolares Lösungsmittel dienen.

Als niedrigsiedendes, mit Wasser mischbares organisches Lösungsmittel für die Extraktion sind beispielsweise Methanol, Ethanol, Tetrahydrofuran und Aceton geeignet. Mit besonderem Vorteil verwendet man Methanol, da dieses in Gegenwart von Wasser praktisch unlöslich in den zur Extraktion verwendeten Kohlenwasserstoffen ist. Im allgemeinen verwendet man das niedrigsiedende, mit Wasser mischbare Lösungsmittel in einem Gewichtsverhältnis von Wasser zu Lösungsmittel von etwa 4:1 bis 1:10.

Eine mögliche vorteilhafte Ausführungsform des erfindungsgemäßen Verfahrens unter Rückführung von Zinkchlorid oder Zinkbromid ist dadurch gekennzeichnet, daß man
im Verfahrensschritt C. die Hauptmenge des Zinkchlorids bzw. Zinkbromids aus der Tocopherol-Lösung mit möglichst wenig Wasser extrahiert und im
Verfahrensschritt D. soviel von der erhaltenen wäßrigen Lösung oder pumpbaren Maische für die folgende Umsetzung in den Umsetzungsprozeß zurückführt, daß nach Ergänzung des noch fehlenden Zinkchlorids bzw. Zinkbromids durch trockenes Zinkchlorid oder Zinkbromid oder eine 85- bis 90 gew.-%ige Lösung oder pumpbare Maische im Reaktionsgemisch für die folgende Umsetzung 1 bis 4, vorzugsweise nicht mehr als 3 mol Wasser pro Zinkchlorid bzw. Zinkbromid vorliegen.

Eine weitere vorteilhafte Ausführungsform des erfindungsgemäßen Verfahrens ist dadurch gekennzeichnet, daß man
im Verfahrensschritt C. durch mehrmaliges Extrahieren mit Wasser oder einem Gemisch aus Wasser und Methanol, dem ggf. etwa 0,5 bis 1 Gew.-% einer Mineralsäure, vorzugsweise HCl, zugesetzt wurde, das Zinkchlorid bzw. Zinkbromid möglichst vollständig aus der Tocopherol-Lösung entfernt und
im Verfahrensschritt D. die erhaltene verdünnte Zinkchlorid- oder Zinkbromid-Lösung durch Abdampfen von Wasser oder Methanol und Wasser so weit aufkonzentriert, daß die Zinkchlorid- bzw. Zinkbromid-Lösung oder pumpbare Maische 1 bis 4 mol Wasser, vorzugsweise nicht mehr als 3,5 mol, insbesondere nicht mehr als 3 mol Wasser, pro mol Zinkchlorid bzw. Zinkbromid enthält und daß man sie so in den Umsetzungsprozeß zurückführt.

Die Zugabe der Mineralsäure zu dem für die Extraktion verwendeten Wasser soll sicherstellen, daß auch bei mehrfacher Extraktion der übliche und vorteilhafte pH-Bereich (kleiner als 5) bestehen bleibt. Mit Vorteil führt man das mehrmalige Extrahieren durch eine sogenannte 1- bis 3-stufige Gegenstromextraktion aus.

Hierbei kann man die erhaltene verdünnte Zinkchlorid- bzw. Zinkbromid-Lösung zunächst in einer speziellen Destillationsvorrichtung in Gegenwart oder Abwesenheit eines geeigneten Azeotropbildners aufkonzentrieren und danach in Form einer ggf. heißen Lösung oder pumpbaren Maische in den Umsetzungsprozeß zurückführen oder aber im Reaktionsgefäß selbst vor der Zugabe von IP in Gegenwart oder Abwesenheit eines geeigneten Azeotropbildners aufkonzentrieren.

Stellt man α-Tocopherol bzw. α-Tocopherylacetat, wie in DE 26 06 830 oder EP 100 471 beschrieben, in Gegenwart von einem Ammmoniumsalz und/oder einem Amin her, dann ist es sogar möglich, daß man im Verfahrensschritt C. nach der Umsetzung das Zinkchlorid bzw. Zinkbromid zusammen mit gegebenenfalls enthaltenem Ammoniumsalz und/oder Amin sowie unumgesetztem TMH mit einer Mischung aus Wasser und Methanol im Verhältnis von etwa 4:1 bis 1:10 aus der erhaltenen Tocopherol-Lösung extrahiert und den erhaltenen Extrakt nach Aufkonzentrieren, in Form einer gegebenenfalls heißen Lösung oder pumpbaren Maische so in den Umsetzungsprozeß zurückführt, daß im Reaktionsgemisch für die folgende Umsetzung 1 bis 4 mol Wasser, vorzugsweise nicht mehr als 3 mol Wasser, pro mol Zinkchlorid bzw. Zinkbromid enthalten sind.

Die Umsetzung des ggf. gemäß DE 26 06 830 mit einem Ammoniumsalz und/oder einem Amin behandelten Isophytols oder Phytols mit TMH erfolgt in an sich bekannter Weise bei Temperaturen zwischen 60 und 200°C, vorzugsweise bei 80 bis 140°C, insbesondere bei 90 bis 110°C, und in den oben beschriebenen Kohlenwasserstoffen als Lösungsmittel. Die Lösungsmittelmenge kann in großen Grenzen variiert werden, sie kann den einfachen bis zum zehnfachen Gewichtsteil des IP's betragen. Die Zinkchlorid- bzw. Zinkbromidmenge kann 0,04 Gewichtsteile vom IP betragen bis zu sehr hohen Mengen von 0,5 Gewichtsteilen und höher, die aber keinen Vorteil bringen.

Als Protonendonatoren können Mineralsäuren, wie konz. Salzsäure, konz. Bromwasserstoffsäure, Schwefelsäure, Phosphorsäure oder Natriumhydrogensulfat verwendet werden. Davon wird Salzsäure bevorzugt.

Ferner kann Toluolsulfonsäure oder Trifluormethansulfonsäure verwendet werden, wie auch Mischungen der genannten Säuren.

Das bei der Reaktion entstehende Wasser kann ausgekreist werden, doch ist die Umsetzung in speziellen Fällen auch ohne Wasserauskreisung durchführbar.

Statt mit Zusatz von wäßriger Salzsäure kann mit Vorteil auch unter Einleiten von Chlorwasserstoffgas gearbeitet werden. Dies hat den Vorteil, daß die Säurekonzentration nicht auf einen zu hohen Wert anwachsen kann, da überschüssiges Salzsäuregas sich aus der Reaktionsmischung verflüchtigt. Eine hochsiedende Säure, wie Schwefelsäure kann dagegen in zu hoher Konzentration die Bildung von Nebenprodukten begünstigen. Bei Umsetzungen unter Einleiten von Chlorwasserstoffgas wird im allgemeinen nur wenig Wasser ausgekreist, da sich hierbei aus dem bei der Reaktion gebildeten Wasser und HCl eine etwa 12 %ige wäßrige, konstant siedende Salzsäure bildet, die viel schwerer als Wasser auskreisbar ist.

Die Umsetzung kann diskontinuierlich, aber auch kontinuierlich durchgeführt werden.

Die Menge des für die Extraktion benötigten Wasser hängt ab von der Menge des eingesetzten Zinkhalogenids, von der Menge des verwendeten Lösungsmittels, von der Menge des während der Umsetzung ausgekreisten Wassers, von der Zahl der Extraktionsstufen sowie von dem gewünschten Extraktionsgrad für das Zinkhalogenid. Sie kann daher in weiten Grenzen variieren. Zur Extraktion von nur der Hauptmenge des Zinkhalogenids genügt im allgemeinen eine einfache Wäsche mit ca. 3 bis 10 Vol.-% Wasser, bezogen auf die organische Phase. Zur vollständigen Extraktion wird 2- bis 6 mal mit je 0,5 bis 10 Vol.-% Wasser oder wäßrigem Methanol, vorzugsweise 2 bis 4 mal mit 1 bis 5 Vol.-% Wasser oder wäßrigem Methanol gewaschen. Besonders vorteilhaft gelingt die vollständige Extraktion in einer dreistufigen Gegenstromextraktion mit 1 bis 3 Volumen% Wasser.

Man kann aber auch das bei der Umsetzung gebildete Wasser enthaltende Zinkchlorid oder Zinkbromid als solches entfernen.

Bei zusätzlicher Zugabe von frischem, trockenem Zinkchlorid bzw. Zinkbromid können auch 60 gew.-%ige Zinkchlorid- oder Zinkbromid-Lösungen zurückgeführt werden. Arbeitet man mit praktisch vollständiger Rückführung des Zinkhalogenids, wobei nur sehr kleine Mengen, d.h. nur etwa 0 bis 3 % an trockenem Zinkchlorid oder Zinkbromid in Form einer 85 bis 90 gew.-%igen Lösung zugesetzt werden, sollten die als Katalysatorlösung eingesetzten Zinkhalogenid-Lösungen oder pumpbaren Maischen mindestens 65 Gew.-% Zinkchlorid bzw. Zinkbromid enthalten, was einer Menge von etwa 4 mol Wasser pro mol Zinkchlorid bzw. Zinkbromid entspricht. Mit Vorteil arbeitet man mit einer Katalysatorlösung oder pumpbaren Maischen, die 70 bis 90 Gew.-%, insbesondere 80 bis 90 Gew.-% Zinkhalogenid enthält, was etwa Mengen von 1 bis 3 mol Wasser, insbesondere etwa 1 bis 2 mol Wasser pro mol Zinkhalogenid entspricht. Je höher der Gehalt an Zinkchlorid oder Zinkbromid in der Mischung ist, desto wichtiger ist es, diese während der Aufbewahrung und/oder während des Zudosierens auf Temperaturen von etwa 50 bis 200°C, vorzugsweise 80 bis 200°C zu halten. Bei Zinkchlorid- oder Zinkbromid-Konzentration von mehr als etwa 90 Gew.-% steigt die Gefahr, daß die Lösung, übersättigte Lösung oder Maische erstarrt, was die Prozeßführung erschweren würde.

Die folgenden Beispiele sollen das erfindungsgemäße Verfahren veranschaulichen. Die Ausbeute-Angaben gelten für den reinen Wirkstoff (Vitamine-E-acetat 100%ig) und sind bezogen auf eingesetztes TMH. Die Reinheit wurde durch Gaschromatographische Analyse (GC) des erhaltenen Produkts bestimmt. Die Färbung des Destillats nach der VEA-Destillation wurde durch Absorptionsspektroskopie (Perkin-Elmer 552; Zellenlänge 1 cm Reinsubstanz; Wellenlänge 420 nm gemessen und die Farbintensität C.I. nach der Formel C.I. = - 0,261 (% Transmittance) + 25,23 berechnet.

### Vergleichsbeispiele 1, 2b und 2g und Beispiele 2a, 2c bis 2f und 2h bis 2j.

In einem 4 l-Rührbehälter aus Glas mit aufgesetztem Rückflußkühler und Wasserabscheider wurden jeweils 1170 g Heptan, 600 g Trimethylhydrochinon (TMH: Reinheit > 94%), 12 g Tridecylamin (TDA), die aus Tabelle 1 ersichtliche Menge x[g] trockenes Zinkchlorid und die aus Tabelle 1 ersichtliche Menge y[g] einer wäßrigen Zinkchloridlösung der Konzentration z [Gew.-%] vorgelegt und der Ansatz unter Rühren aufgeheizt. Anschließend wurde Chlorwasserstoff (ca. 20 g/h) zudosiert. In die siedende Mischung wurden innerhalb von 1 Stunde (h) unter Auskreisen von Wasser (W) jeweils 1210 g Isophytol (IP; 98 %ig) zudosiert. Anschließend ließ man noch 1 h bei Siedetemperatur nachreagieren. Dann wurde mit 100 ml W gewaschen. Bei dreimaligem Waschen mit je 100 ml W können 98 bis 100 % des eingesetzten Zinkchlorids zurückgewonnen werden. Anschließend wurde mit wäßrigem Methanol gewaschen. Nach Abziehen des Lösungsmittels wurde das rohe α-Tocopherol mit Essisäureanhydrid durch Erhitzen unter Rückfluß verestert und das erhaltene Vitamin-E-acetat (VEA) in der aus Tabelle 1 ersichtlichen Ausbeute an reinem Vitamin-E-acetat, bezogen auf eingesetztes TMH, und Reinheit (bestimmt durch GC) erhalten.

**Tabelle 1**

| Bsp. | Menge x ZnCl₂ trocken [g] | Menge y ZnCl₂-Lösung [g] | Konz. z der ZnCl₂L-Lösung [Gew.-%] | Eintrag Wasser [mol/mol ZnCl₂] | Ausbeute [% der Theorie] | Reinheit [%] |
|---|---|---|---|---|---|---|
| 1* | 125 | - | - | 0 | 97 | 97,7 |
| 2a | 100 | 68 | 56 | 1,6 | 95 | 97,5 |
| 2b* | 28 | 197 | 56 | 4,8 | 88 | 94,9 |
| 2c | 25 | 125 | 80 | 1,4 | 96 | 97,3 |
| 2d | - | 156 | 80 | 1,9 | 95 | 97,1 |
| 2e | - | 313 | 80 | 1,9 | 96 | 97,4 |
| 2f | 100 | 31 | 80 | 0,4 | 98 | 97,8 |
| 2g* | 250 | - | - | 0 | 96 | 96,7 |
| 2h | - | 175 | 72 | 3,0 | 97 | 97 |
| 2i | - | 191 | 65 | 4,0 | 93 | 95,5 |
| 2j | - | 764 | 65 | 4,0 | 90 | 96,0 |

| | | | | | | |
|---|---|---|---|---|---|---|
| * Vergleichsbeispiele | | | | | | |

Die Beispiele 2a, 2c bis 2f und 2h bis 2j zeigen den Einfluß von W auf die Katalysatoraktivität. Bei Mitverwendung von bis zu 3 mol Wasser pro mol ZnCl₂ werden keine signifikanten Verschlechterungen der Ausbeute und der Reinheit beobachtet, ab 4 mol W pro mol ZnCl₂ sinken die Ausbeuten leicht ab. Beispiele 2e und Vergleichsbeispiel 2g zeigen, daß größere Katalysatormengen keinen Vorteil mehr bringen.

### Beispiel 3

### Umsetzung in Gegenwart von wenig trockenem ZnCl₂ und stark verdünnter ZnCl₂-Lösung

In einem 4 l-Rührbehälter aus Glas mit aufgesetztem Rückflußkühler und a) mit Wasserabscheider und b) ohne Wasserabscheider wurden jeweils 1170 g Heptan, 600 g TMH, 12 g TDA und 23 g trokkenes ZnCl₂ sowie 463 g einer wäßrigen ZnCl₂-Lösung (Konzentration: 20 Gew.-%; Verhältnis von W/ZnCl₂ = 24 mol/mol) vorgelegt und der Ansatz unter Rühren und a) unter Abscheiden von W und b) ohne Abscheidung von W aufgeheizt und ca. 20 g/h Chlorwasserstoff zudosiert. In die siedende Mischung wurden innerhalb von 1 h 1210 g IP zudosiert und das Reaktionsgemisch noch 2 h nachreagieren lassen.

Bei Reaktionsansatz a) setzte die Umsetzung zwischen TMH und IP ein nachdem der überwiegende Teil des Wassers aus der ZnCl₂-Lösung sich im Wasserabscheider abgeschieden hatte. Der Ansatz wurde analog Beispiel 2 aufgearbeitet.

Im Reaktionsansatz b) wurde das gewünschte α-Tocopherol nur in Spuren gebildet, was zeigt, daß größere Mengen an W im Reaktionsgemisch den Katalysator völlig desaktivieren.

Die jeweils erzielten Ausbeuten sind in Tabelle 2 dargestellt. Wie Tabelle 2 zeigt, sind die Ausbeute und die Reinheit des erhaltenen Tocopherols schlechter als in Beispiel 4, was zeigt, daß es vorteilhafter ist, wenn man die Katalysatorlösung bereits vor Beginn der Umsetzung aufkonzentriert.

**Tabelle 2**

| Bsp. | Menge x ZnCl₂ trocken [g] | Menge y ZnCl₂-Lösung [g] | Konz. z der ZnCl₂-Lösung [Gew. -%] | Eintrag W [mol W/mol ZnCl₂] | Ausbeute [% der Theorie] | Reinheit [%] |
|---|---|---|---|---|---|---|
| 3a* | 23 | 463 | 20 | 24 | 86 | 93,5 |
| 3b** | 23 | 463 | 20 | 24 | <5 | |

| | | | | | | |
|---|---|---|---|---|---|---|
| * = mit Auskreisen von W | | | | | | |
| ** = ohne Auskreisen von W | | | | | | |

### Beispiel 4

### Umsetzung in Gegenwart von wenig trockenem ZnCl₂ und einer konzentrierten ZnCl₂-Lösung

In dem oben beschriebenen 4 l-Rührbehälter wurden jeweils 1170 g Heptan, 600 g TMH, 12 g TDA, 23,2 g trockenes ZnCl₂ und 115,9 g einer 80 gew.-%igen ZnCl₂-Lösung vorgelegt und der Ansatz a) unter Auskreisen von W und b) ohne Auskreisen von W aufgeheizt. Sodann wurden ca. 20 g/h Chlorwasserstoff zudosiert und in die siedende Mischung innerhalb von 1 h 1210 g IP zudosiert. Anschließend ließ man 1 h nachreagieren. Anschließend wurde einmal mit 300 ml W und 3x mit wäßrigem Methanol gewaschen und analog Beispiel 2 mit Acetanhydrid verestert.

Die erzielten Ausbeuten und Reinheiten sind in Tabelle 3 dargestellt:

**Tabelle 3**

| Bsp. | ZnCl₂ trocken [g] | Menge y ZnCl₂-Lösung [g] | Konz. z der ZnCl₂-Lösung [Gew.-%] | Eintrag W [mol W/mol ZnCl₂] | Ausbeute [% der Theorie] | Reinheit [%] |
|---|---|---|---|---|---|---|
| 4a* | 23,2 | 115,9 | 80 | 1,5 | 97 | 97,3 |
| 4b** | 23,2 | 115,9 | 80 | 1,5 | 97 | 97,5 |

| | | | | | | |
|---|---|---|---|---|---|---|
| * = mit Auskreisen von W | | | | | | |
| ** = ohne Auskreisen von W | | | | | | |

Die Beispiele 4a und 4b zeigen, daß bei einem Wassereintrag von nur 1,5 mol/mol ZnCl₂ das Auskreisen von W ohne Verschlechterung von Ausbeute und Reinheit entfallen kann.

### Beispiel 5

### Mehrfache Rückführung von ZnCl₂; mittlerer Rückführungsgrad 78 %.

In dem oben beschriebenen 4 l-Rührbehälter mit Rückflußkühler und Wasserabscheider wurden 1170 g Heptan, 600 g TMH, 12 g TDA und die aus Tabelle 4 ersichtliche Menge x [g] trockenes ZnCl₂ und die aus Tabelle 4 ersichtliche Menge y [g] ZnCl₂-Lösung der aus Tabelle 4 ersichtlichen Konzentration z vorgelegt und der Ansatz unter Rühren aufgeheizt. Sodann wurde ca. 20 g/h Chlorwasserstoffgas zudosiert, in die siedende Mischung innerhalb von 1 h 1210 g IP zudosiert und anschließend das Reaktionsgemisch noch 1 h nachreagieren lassen.

Anschließend wurde durch einmalige Zugabe von 100 ml W ein Großteil (ca. 80%) des ZnCl₂ ausgewaschen. Die Heptan-Tocopherol-Phase wurde mit wäßrigem Methanol gewaschen, eingedampft und das erhaltene rohe Tocopherol mit Essigsäureanhydrid verestert.

Die erhaltene wäßrige ZnCl₂-Lösung (ca. 200 g mit einer mittleren Konzentration von ca. 50 Gew.-% ZnCl₂) wurde in einem Sambay-Verdampfer zu der aus Tabelle 4 ersichtlichen Konzentration z aufkonzentriert. Das Konzentrat wurde bei 100°C gelagert und erstarrte bei diesen Temperaturen nicht. Es wurde bei dem nächsten Kondensationsansatz neben der aus Tabelle 4 ersichtlichen Menge an trockenem ZnCl₂ wieder als Katalysator eingesetzt. Auf gleiche Weise wurde jeweils das zurückgewonnene ZnCl₂ in 10 aufeinanderfolgenden Kondensationsansätzen eingesetzt. Der Eintrag von W betrug bei allen Ansätzen etwa 1,0 bis 1,2 mol W pro mol ZnCl₂. In Tabelle 4 sind die Reaktionsbedingungen sowie Ausbeuten und Reinheiten des mit zurückgeführtem ZnCl₂ erhaltenen Vitamin-E-acetats angegeben.

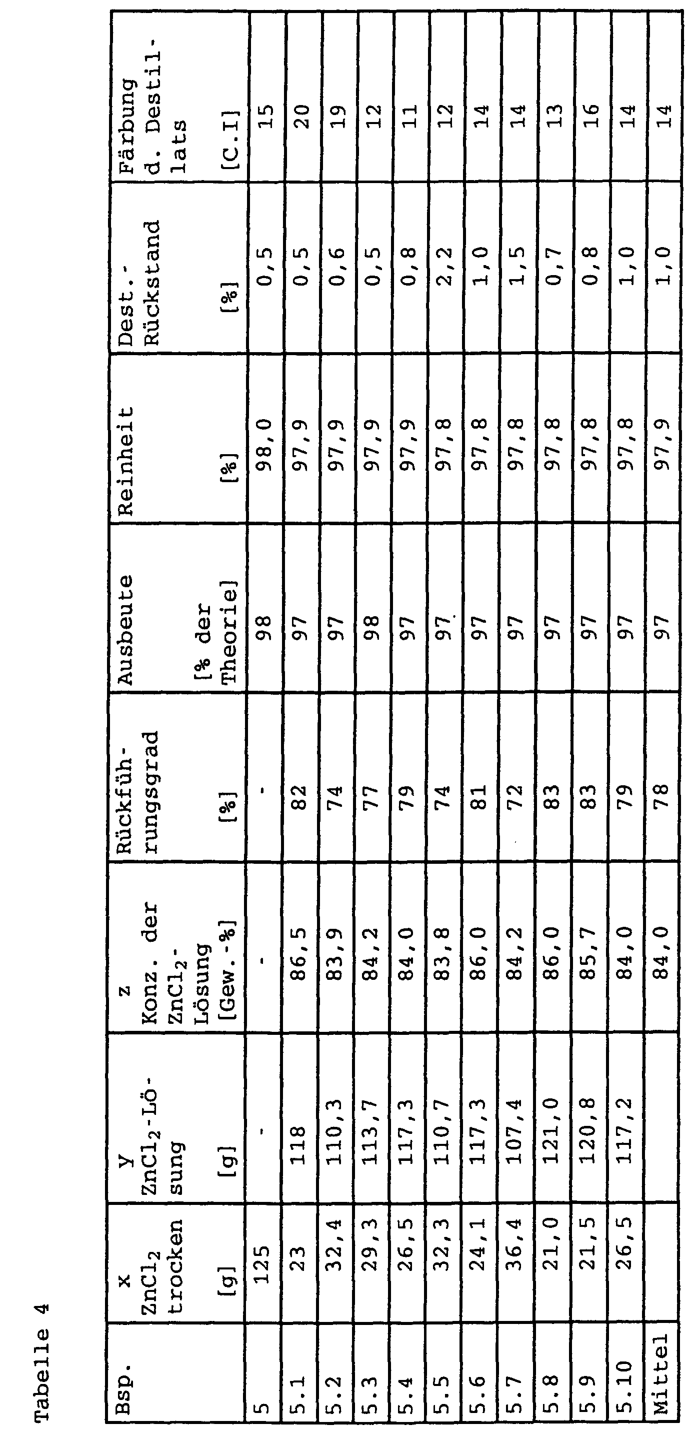

Die Beispiele 5 bis 5.10 zeigen, daß selbst nach 10-maligem Rückführen von ZnCl₂-Lösung der angegebenen Konzentration in Folge keine Verschlechterung der Ausbeute oder der Reinheit des hergestellten VEA's beobachtet wurde. Auch die Bildung hochsiedender Nebenkomponenten, die bei einer Vakuumdestillation des rohen Vitamin-E-acetats als nichtverdampfbarer Rückstand anfallen sowie die Färbung des Destillats (angegeben als Colour Index C.I.) blieben ohne signifikante Änderung.

### Beispiel 6

### Vielfache Rückführung von ZnCl₂; mittlerer Rückführungsgrad > 97 %.

Analog Beispiel 5 wurden 600 g TMH in Gegenwart von der aus Tabelle 5 ersichtlichen Menge x [g] an trockenem ZnCl₂ und y [g] einer zurückgeführten wäßrigen ZnCl₂-Lösung mit einer Konzentration von Z Gew.-% mit 1210 g IP zu α-Tocopherol umgesetzt. Nach 1-stündigem Nachreagieren wurde dreimal mit je 100 ml Wasser gewaschen, wobei 98-100 % des eingesetzten ZnCl₂ zurückerhalten wurden. Nach Abziehen des Lösungsmittels wurde das rohe α-Tocopherol mit Acetanhydrid verestert.

So wurden jeweils etwa 400 bis 420 g ZnCl₂-Lösung mit einer mittleren Konzentration von 30 Gew.-% ZnCl₂ erhalten. Diese Lösungen wurden filtriert und an einem Sambay-Verdampfer aufkonzentriert. Das Konzentrat wurde bis zur Wiederverwendung bei 100°C gelagert, wobei es nicht erstarrte. Es wurde, wie oben beschrieben, jeweils für den folgenden Kondensationsansatz als Katalysator verwendet.

Die Beispiele 6 bis 6.20 zeigen, daß selbst nach 20-maligem Rückführen von ZnCl₂-Lösung der in Tabelle 5 angegebenen Konzentration z weder eine Verschlechterung der Ausbeute und der Reinheit des VEA's beobachtet werden konnte, noch der Anteil an nichtverdampfbarem Rückstand bei der VEA-Destillation oder die Färbung des Destillats anstieg.

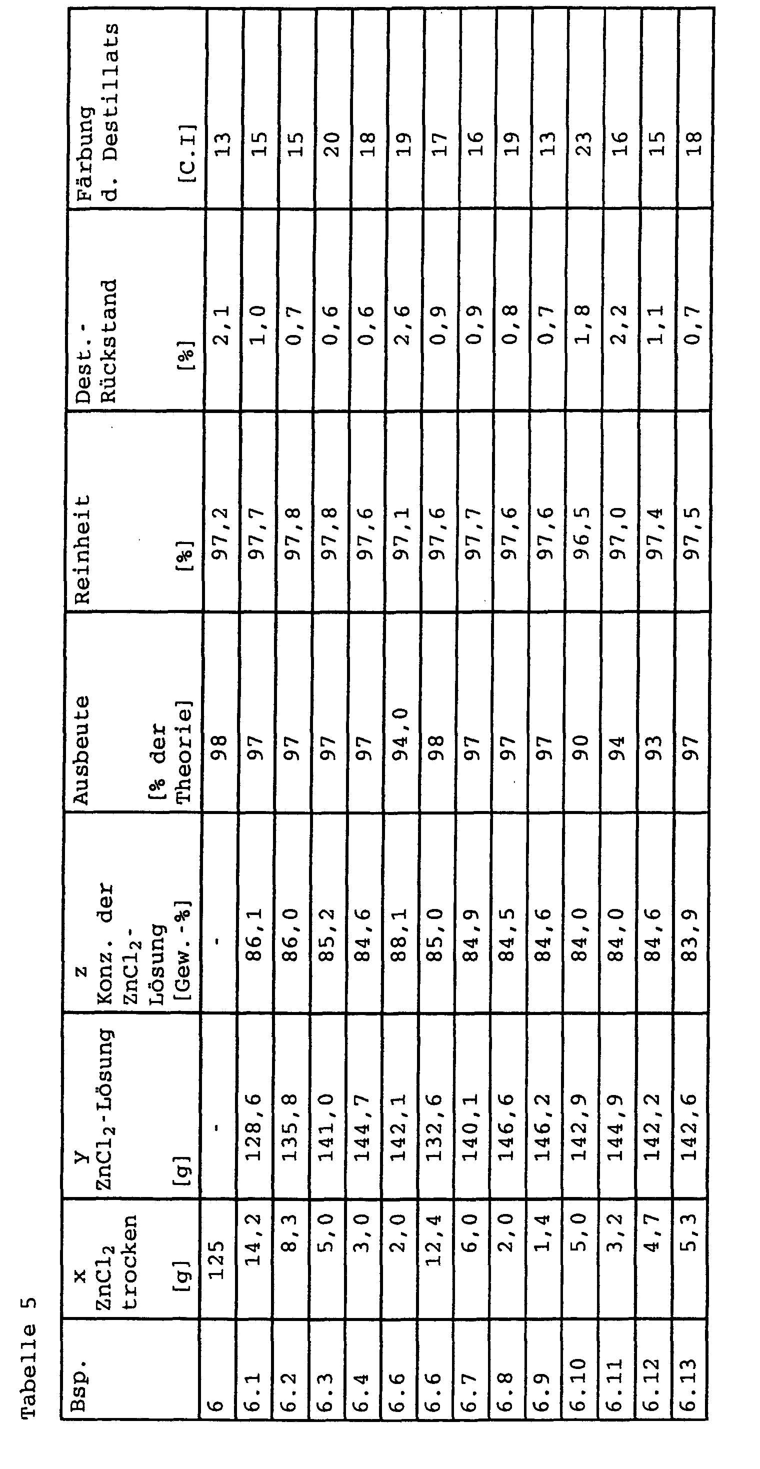

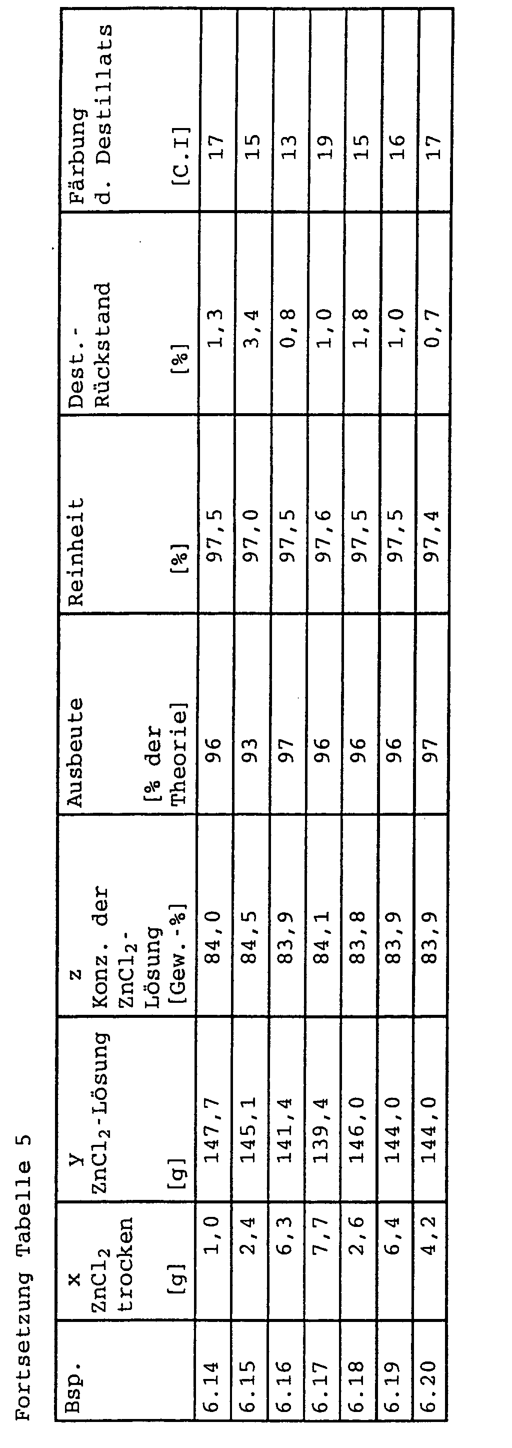

### Beispiel 7

### Rückführung von ZnCl₂ mit Aufkonzentrierung der Katalysatorlösung durch Azeotrop-Destillation im Reaktionsgefäß

In einem 0,5 l-Dreihalskolben aus Glas mit Blattrührer, Tropftrichter, Rückflußkühler und Wasserabscheider wurden 150 g Heptan, 76 g TMH, 1,5 g TDA und 16 g trockenes ZnCl₂ vorgelegt und der Ansatz unter Rühren aufgeheizt. Chlorwasserstoff wird zudosiert (ca. 5 g/h). In die siedende Mischung wurden 153 g IP zudosiert.

Man ließ 1 h nachreagieren. Nach dem Abkühlen wurden 12,5 ml Wasser zugesetzt, durchmischt und nach dem Absetzenlassen die Phasen getrennt. Die heptanische Phase wurde eingeengt und das rohe Tocopherol mit Essigsäureanhydrid durch Erhitzen unter Rückfluß zu VEA verestert.

Die wäßrige Phase wurde für den nächsten Versuch als Katalysator vorgelegt. 150 g Heptan, 76 g TMH und 1,6 g frisches Zinkchlorid wurden zugegeben und der Ansatz wie oben unter Rühren aufgeheizt. Das W wurde durch Azeotrop-Destillation mit dem Heptan am Wasserabscheider ausgekreist und Chlorwasserstoff zudosiert (ca. 5 g/h). Nachdem ca. 10 ml des Wassers ausgekreist worden waren, wurden in die siedende Mischung 153 g IP zudosiert. Man ließ nachreagieren. Die Aufarbeitung wurde wie oben beschrieben durchgeführt.

Auf diese Art wurde das ZnCl₂ dreimal zurückgeführt. In allen Ansätzen wurden Ausbeuten von 97 % der Theorie und Reinheiten von 97,7 % erzielt.

### Beispiel 8

### Rückführung von Zinkchlorid sowie des Phasentransferkatalysators TDA-HCl durch Extraktion mit W/Methanol

In einem 0,5 l-Dreihalskolben aus Glas mit Blattrührer, Tropftrichter, Rückflußkühler und Wasserabscheider wurden 150 g Heptan, 76 g TMH, 1,5 g TDA und 16 g trockenes ZnCl₂ vorgelegt und der Ansatz unter Rühren aufgeheizt. Es wurde Chlorwasserstoff zudosiert (ca. 5 g/h) und in die siedende Mischung 153 g IP zudosiert. Man ließ 1 h nachreagieren. Nach dem Abkühlen wurden dreimal je 100 ml W und Methanol zugesetzt, das Reaktionsgemisch durchmischt und nach dem Absetzenlassen die Phasen getrennt. Die heptanische Phase wurde eingeengt und das rohe Tocopherol durch Erhitzen unter Rückfluß mit Essigsäureanhydrid zu VEA verestert.

Die wäßrig-methanolische Phase wurde eingedampft und für den nächsten Versuch als Katalysator vorgelegt. 150 g Heptan, 76 g TMH und 1,6 g frisches ZnCl₂ wurden zugegeben, der Ansatz wie oben beschrieben unter Rühren aufgeheizt und Chlorwasserstoff zudosiert (ca. 5 g/h). In die siedende Mischung wurden 153 g IP zudosiert. Man ließ 1 h nachreagieren. Die Aufarbeitung wurde wie oben beschrieben durchgeführt.

Auf diese Art wurde das Zinkchlorid und TDA dreimal zurückgeführt. Alle Ansätze lieferten Ausbeuten an VEA von etwa 97 % der Theorie und Reinheiten von 97,7 %.

Dieses Beispiel zeigt, daß durch Extraktion mit einem Gemisch aus W und Methanol im Verhältnis von etwa 1:1 nicht nur das Zinkchlorid, sondern auch der Phasentransferkatalysator TDA.HCl nahezu vollständig in den Prozeß zurückgeführt werden kann.

### Beispiel 9

### Vielfache Rückführung von Zinkbromid.

Analog Beispiel 5 - jedoch unter Zugabe von 22 mol einer 47 Gew.-%igen wässrigen HBr-Lösung anstelle des Einleitens von HCl-Gas - wurden 600 g TMH in Gegenwart von der aus Tabelle 6 ersichtlichen Menge x [g] an trockenem Zinkbromid und y [g] einer zurückgeführten wäßrigen Zinkbromid-Lösung mit einer Konzentration von Z Gew.-% mit 1210 g IP zu α-Tocopherol umgesetzt. Nach 1-stündigem Nachreagieren wurde dreimal mit je 100 ml Wasser gewaschen, wobei 98-100 % des eingesetzten Zinkbromids zurückerhalten wurden. Nach Abziehen des Lösungsmittels wurde das rohe α-Tocopherol mit Acetanhydrid verestert.

So wurden jeweils etwa 460 bis 480 g Zinkbromid-Lösung mit einer mittleren Konzentration von 37 Gew.-% Zinkbromid erhalten. Diese Lösungen wurden filtriert und an einem Sambay-Verdampfer aufkonzentriert. Das Konzentrat wurde bis zur Wiederverwendung bei 100°C gelagert, wobei es nicht erstarrte. Es wurde, wie oben beschrieben, jeweils für den folgenden Kondensationsansatz als Katalysator verwendet.

Die Beispiele 9 bis 9.2 zeigen, daß bei dem erfindungsgemäßen Verfahren auch bei Verwendung von Zinkbromid die Rückführung des Katalysators ohne Verschlechterung der Ausbeute oder der Reinheit des Vitamin-E-acetats möglich ist.

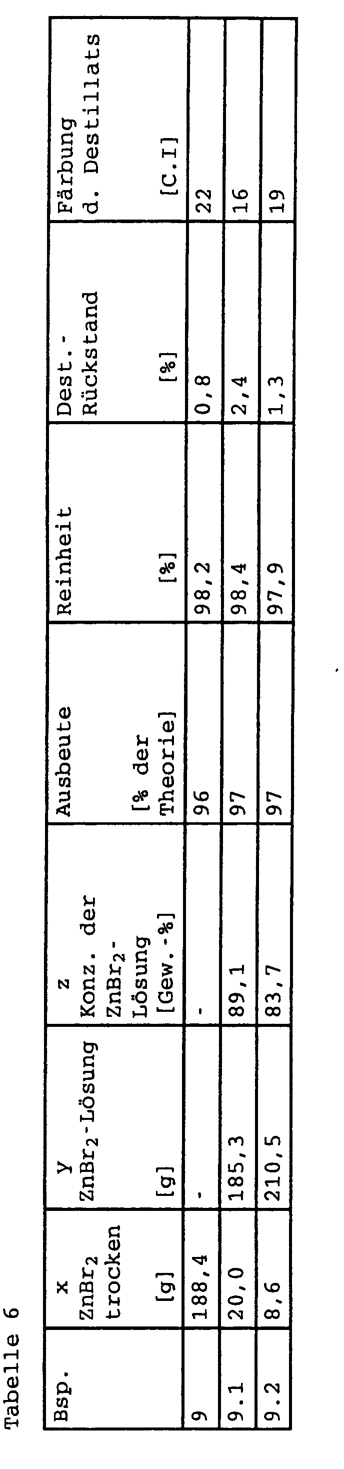

## Patentansprüche

1. Verfahren zur Herstellung von α-Tocopherol oder α-Tocopherylacetat durch Umsetzen von 2,3,5-Trimethyl-hydrochinon mit Phytol oder Isophytol in Gegenwart von einem Zinkhalogenid-Kondensationskatalysator, ausgewählt aus der Gruppe Zinkchlorid oder Zinkbromid, Mischungen derselben, sowie Mischungen derselben mit basischen Chloriden und Bromiden des Zinks und einem Protonendonator in einem Lösungsmittel und ggf. anschließende Veresterung mit Acetanhydrid, **dadurch gekennzeichnet, daß** man
A. Die Umsetzung in einem mit Wasser nicht oder nur wenig mischbaren unpolaren Lösungsmittel durchführt und
B. das benötigte Zinkhalogenid in Form einer Mischung aus 1 bis 4 mol Wasser pro mol Zinkhalogenid in die Umsetzung einbringt.

2. Verfahren nach Anspruch 1, **dadurch gekennzeichnet, daß** man
C. nach erfolgter Umsetzung das Zinkhalogenid abtrennt und/oder mit Wasser oder einem Gemisch aus Wasser und einem niedrigsiedenden, mit Wasser mischbaren organischen Lösungsmittel aus der erhaltenen Tocopherol-Lösung extrahiert und
D. die erhaltene Zinkhalogenid-Lösung ggf. nach Aufkonzentrieren in Form einer 60 bis 90 gew.-%igen gegebenenfalls heißen Lösung oder pumpbaren Maische teilweise oder vollständig so in den Umsetzungsprozeß zurückführt, daß im Reaktionsgemisch nach Ergänzen des noch fehlenden Zinkhalogenids für die folgende Umsetzung 1 bis 4 mol Wasser, pro mol Zinkhalogenid vorliegen.

3. Verfahren zur Herstellung von α-Tocopherol oder α-Tocopherylacetat durch Umsetzen von 2,3,5-Trimethyl-hydrochinon mit Isophytol oder Phytol in Gegenwart von einem Zinkhalogenid-Kondensationskatalysator, ausgewählt aus der Gruppe Zinkchlorid oder Zinkbromid, Mischungen derselben, sowie Mischungen derselben mit basischen Chloriden und Bromiden des Zinks und einem Protonendonator in einem Lösungsmittel und gegebenenfalls anschließende Veresterung mit Acetanhydrid, das **dadurch gekennzeichnet ist, daß** man
A, die Umsetzung in einem mit Wasser nicht oder nur wenig mischbaren unpolaren Lösungsmittel durchführt,
C. nach erfolgter Umsetzung das Zinkhalogenid abtrennt und/oder mit Wasser oder einem Gemisch aus Wasser und einem niedrigsiedenden, mit Wasser mischbaren organischen Lösungsmittel aus der erhaltenen Tocopherol-Lösung extrahiert und
D. die erhaltene Zinkhalogenid-Lösung ggf. nach Aufkonzentrieren in Form einer 60 bis 90 gew.-%igen gegebenenfalls heißen Lösung oder pumpbaren Maische teilweise oder vollständig so in den Umsetzungsprozeß zurückführt, daß im Reaktionsgemisch nach Ergänzen des noch fehlenden Zinkhalogenids für die folgende Umsetzung 1 bis 4 mol Wasser, pro mol Zinkhalogenid vorliegen.

4. Verfahren gemäß Anspruch 1 oder 2, **dadurch gekennzeichnet, daß** man die Umsetzung in Hexan, Heptan, Cyclohexan, Octan, Nonan, Decan, Toluol, Xylol oder in einem Gemisch von 2 oder mehreren dieser Lösungsmittel als mit Wasser nicht oder nur wenig mischbarem unpolaren Lösungsmittel durchführt.

5. Verfahren gemäß Anspruch 2 oder 3, **dadurch gekennzeichnet, daß** man
im Verfahrensschritt C. die Hauptmenge des Zinkhalogenids aus der Tocopherol-Lösung mit möglichst wenig Wasser oder möglichst wenig eines Gemisches aus Wasser und Methanol extrahiert und
im Verfahrensschritt D. soviel von der erhaltenen wäßrigen Lösung oder pumpbaren Maische in den Umsetzungsprozeß zurückführt, daß nach Ergänzung des noch fehlenden Zinkhalogenids durch trockenes Zinkhalogenid oder eine 85 bis 90 gew.-%ige Lösung oder pumpbare Maische im Reaktionsgemisch nicht mehr als 3 mol Wasser pro mol Zinkhalogenid vorliegen.

6. Verfahren gemäß Anspruch 2 oder 3, **dadurch gekennzeichnet, daß** man
im Verfahrensschritt C. durch mehrmaliges Extrahieren mit Wasser oder einem Gemisch aus Wasser und Methanol, dem ggf. etwa 1 Gew.-% HCl zugesetzt wurde, das Zinkhalogenid möglichst vollständig aus der Tocopherol-Lösung entfernt und
im Verfahrensschritt D. die erhaltene verdünnte Zinkhalogenid-Lösung durch Abdampfen von Wasser oder Methanol und Wasser so weit aufkonzentriert, daß diese nicht mehr als 3 mol Wasser pro mol Zinkhalogenid enthält und daß man sie so in den Umsetzungsprozeß zurückführt.

7. Verfahren gemäß Anspruch 6, **dadurch gekennzeichnet, daß** man die erhaltene verdünnte Zinkhalogenid-Lösung zunächst in einer speziellen Destillationsvorrichtung in Gegenwart oder Abwesenheit eines geeigneten Azeotropbildners aufkonzentriert und danach in Form einer ggf. heißen Lösung oder pumpbaren Maische in den Umsetzungsprozeß zurückführt.

8. Verfahren gemäß Anspruch 6, **dadurch gekennzeichnet, daß** man die erhaltene verdünnte Zinkhalogenid-Lösung im Reaktionsgefäß selbst vor der Zugabe von Isophytol in Gegenwart oder Abwesenheit des für die Umsetzung verwendeten Lösungsmittels aufkonzentriert.

9. Verfahren gemäß Anspruch 2 oder 3, **dadurch gekennzeichnet, daß** man
im Verfahrensschritt C nach erfolgter Umsetzung das Zinkhalogenid zusammen mit gegebenenfalls enthaltenem Ammoniumsalz und/oder Amin und unumgesetztem Trimethylhydrochinon mit einer Mischung aus Wasser und Methanol im Gewichtsverhältnis von etwa 4:1 bis 1:10 aus der erhaltenen Tocopherol-Lösung extrahiert und den erhaltenen Extrakt nach Aufkonzentrieren in Form einer gegebenenfalls heißen Lösung oder pumpbaren Maische so in den Umsetzungsprozeß zurückführt, daß im Reaktionsgemisch nach Ergänzen des noch fehlenden Zinkhalogenids für die folgende Umsetzung nicht mehr als 3 mol Wasser pro mol Zinkhalogenid enthalten sind.

## Claims

1. A process for preparing α-tocopherol or α-tocopheryl acetate by reacting 2,3,5-trimethylhydroquinone with phytol or isophytol in the presence of a zinc halide condensation catalyst, selected from the group consisting of zinc chloride or zinc bromide, mixtures thereof and mixtures thereof with basic chlorides and bromides of zinc, and a proton donor in a solvent with or without subsequent esterification with acetic anhydride, which comprises
A. carrying out the reaction in a nonpolar solvent which is only slightly water-miscible, if at all and
B. introducing the required zinc halide into the reaction in the form of a mixture of from 1 to 4 mol of water per mol of zinc halide.

2. A process as claimed in claim 1, which comprises
C. after completing the reaction, separating off the zinc halide and/or extracting it from the resulting tocopherol solution with water or a mixture of water and a low-boiling water-miscible organic solvent and
D. partly or completely recycling the zinc halide solution obtained, with or without concentration, in the form of a from 60 to 90% strength by weight possibly hot solution or pumpable magma to the reaction process in such a manner that, after making up the zinc halide still missing, from 1 to 4 mol of water per mol of zinc halide are present in the reaction mixture for the following reaction.

3. A process for preparing α-tocopherol or α-tocopheryl acetate by reacting 2,3,5-trimethylhydroquinone with phytol or isophytol in the presence of a zinc halide condensation catalyst and a proton donor in a solvent with or without subsequent esterification with acetic anhydride, which comprises
A. carrying out the reaction in a nonpolar solvent which is only slightly water-miscible, if at all,
C. after completing the reaction, separating off the zinc halide and/or extracting it from the resulting tocopherol solution with water or a mixture of water and a low-boiling water-miscible organic solvent and
D. partly or completely recycling the zinc halide solution obtained, with or without concentration, in the form of a from 60 to 90% strength by weight possibly hot solution or pumpable magma to the reaction process in such a manner that, after making up the zinc halide still missing, from 1 to 4 mol of water per mol of zinc halide are present in the reaction mixture for the following reaction.

4. A process as claimed in claim 1 or 2, wherein the reaction is carried out in hexane, heptane, cyclohexane, octane, nonane, decane, toluene, xylene or in a mixture of 2 or more of these solvents as the nonpolar solvent which is only slightly water-miscible if at all.

5. A process as claimed in claim 2 or 3, wherein
in process step C. the majority of the zinc halide is extracted from the tocopherol solution using very little water or very little of a mixture of water and methanol and
in process step D. sufficient of the resulting aqueous solution or pumpable magma is recycled to the reaction process so that, after making up the zinc halide still missing using anhydrous zinc halide or a from 85 to 90% strength by weight solution or pumpable magma, not more than 3 mol of water per mol of zinc halide are present in the reaction mixture.

6. A process as claimed in claim 2 or 3, wherein
in process step C. the zinc halide is removed as completely as possible from the tocopherol solution by repeated extraction with water or a mixture of water and methanol with or without about 1% by weight of added HCl and
in process step D. the resulting dilute zinc halide solution is concentrated by evaporating water or methanol and water to the extent that these contain no more than 3 mol of water per mol of zinc halide and it is recycled in this state to the reaction process.

7. A process as claimed in claim 6, wherein the resulting dilute zinc halide solution is first concentrated in a special distillation apparatus in the presence or absence of a suitable azeotrope former and then in the form of a possibly hot solution of pumpable magma it is recycled to the reaction process.

8. A process as claimed in claim 6, wherein the resulting dilute zinc halide solution is concentrated in the reaction vessel itself prior to the addition of isophytol in the presence or absence of the solvent used for the reaction.

9. A process as claimed in claim 2 or 3, wherein,
in process step C after completing the reaction, the zinc halide, together with any ammonium salt and/or amine and unreacted trimethylhydroquinone present is extracted from the resulting tocopherol solution using a mixture of water and methanol in a weight ratio of from about 4:1 to 1:10 and the resulting extract, after concentration in the form of a possibly hot solution or pumpable magma is recycled to the reaction process in such a manner that, in the reaction mixture, after making up the zinc halide still missing, not more than 3 mol of water per mol of zinc halide are present for the following reaction.

## Revendications

1. Procédé pour la préparation de l'alpha-tocophérol ou de l'acétate d'alpha-tocophéryle par réaction de la 2,3,5-triméthyl-hydroquinone avec le phytol ou l'isophytol en présence d'un catalyseur de condensation à base d'halogénure de zinc choisi dans le groupe consistant en le chlorure ou le bromure de zinc, leurs mélanges et leurs mélanges avec des chlorures et bromures basiques du zinc et un donneur de protons dans un solvant, suivie le cas échéant d'une estérification par l'anhydride acétique, **caractérisé par le fait que**
A. la réaction est réalisée dans un solvant non polaire non miscible ou peu miscible à l'eau et
B. on introduit l'halogénure de zinc nécessaire dans la réaction sous la forme d'un mélange d'1 à 4 mol d'eau par mol d'halogénure de zinc.

2. Procédé selon la revendication 1, **caractérisé par le fait que**
C. après la réaction, on sépare l'halogénure de zinc et/ou on l'extrait de la solution de tocophérol ainsi obtenue par l'eau ou par un mélange d'eau et d'un solvant organique à bas point d'ébullition, miscible à l'eau et
D. on recycle la solution d'halogénure de zinc ainsi obtenue en totalité ou en partie dans les opérations, éventuellement après concentration sous la forme d'un solution ou d'une bouillie pompable, à une concentration de 60 à 90 % en poids, éventuellement chauffée, en quantité telle que dans le mélange de réaction, après addition du complément d'halogénure de zinc nécessaire pour la réaction subséquente, il y ait 1 à 4 mol d'eau par mol de l'halogénure de zinc.

3. Procédé pour la préparation de l'alpha-tocophérol ou de l'acétate d'alpha-tocophéryle par réaction de la 2,3,5-triméthyl-hydroquinone avec l'isophytol ou le phytol en présence d'un catalyseur de condensation à base d'halogénure de zinc choisi dans le groupe consistant en le chlorure de zinc ou le bromure de zinc, leurs mélanges, et leurs mélanges avec des chlorures et bromures basiques du zinc et un donneur de protons dans un solvant, éventuellement suivie d'une estérification par l'anhydride acétique, ce procédé se caractérisant par le fait que
A. on réalise la réaction dans un solvant non polaire, non miscible ou peu miscible à l'eau,
C. après la réaction, on sépare l'halogénure de zinc et/ou on l'extrait de la solution de tocophérol ainsi obtenue par l'eau ou par un mélange d'eau et d'un solvant organique à bas point d'ébullition, miscible à l'eau, et
D. on recycle la solution d'halogénure de zinc ainsi obtenue en totalité ou en partie dans les opérations, éventuellement après concentration, sous la forme d'une solution ou d'un mélange pompable à une concentration de 60 à 90 % en poids, éventuellement chauffé, en quantité telle que, dans le mélange de réaction, après addition du complément d'halogénure de zinc nécessaire pour la réaction subséquente, il y ait 1 à 4 mol d'eau par mol d'halogénure de zinc.

4. Procédé selon la revendication 1 ou 2, **caractérisé par le fait que** l'on réalise la réaction dans l'hexane, l'heptane, le cyclohexane, l'octane, le nonane, le décane, le toluène, le xylène ou dans un mélange de deux ou plusieurs de ces solvants, qui constitue le solvant non polaire, non miscible ou peu miscible à l'eau.

5. Procédé selon la revendication 2 ou 3, **caractérisé par le fait que**
au stade opératoire C. on extrait la plus grande partie de l'halogénure de zinc de la solution de tocophérol par de l'eau en quantité aussi faible que possible ou par un mélange d'eau et de méthanol en quantité aussi faible que possible et
au stade opératoire D. on recycle la solution ou la bouillie pompable aqueuse ainsi obtenue dans les opérations en quantité telle qu'après addition du complément nécessaire d'halogénure de zinc sous forme d'halogénure de zinc sec ou d'une solution ou bouillie pompable à une concentration de 85 à 90 % en poids, il n'y ait pas plus de 3 mol d'eau par mol d'halogénure zinc dans le mélange de réaction.

6. Procédé selon la revendication 2 ou 3, **caractérisé par le fait que**
au stade opératoire C. par extraction répétée à l'aide d'eau ou d'un mélange d'eau et de méthanol additionné le cas échéant d'environ 1 % en poids d' HCl, on élimine l'halogénure de zinc aussi complètement que possible de la solution de tocophérol et
au stade opératoire D. on concentre la solution diluée d'halogénure de zinc ainsi obtenue par évaporation de l'eau ou du méthanol et de l'eau dans une mesure suffisante pour que cette solution ne contienne pas plus de 3 mol d'eau par mol d'halogénure de zinc, et on la recycle alors dans les opérations.

7. Procédé selon la revendication 6, **caractérisé par le fait que** l'on concentre d'abord la solution diluée d'halogénure de zinc ainsi obtenue dans un dispositif spécial de distillation en présence ou en l'absence d'un agent d'entrainement azéotropique approprié puis on la recycle dans les opérations sous la forme d'une solution ou d'une bouillie pompable éventuellement chauffée.

8. Procédé selon la revendication 6, **caractérisé par le fait que** l'on concentre la solution diluée d'halogénure de zinc ainsi obtenue dans le récipient de réaction lui-même, avant l'addition de l'isophytol, en présence ou en l'absence du solvant utilisé dans la réaction.

9. Procédé selon la revendication 2 ou 3, **caractérisé par le fait que**
au stade opératoire C. après la réaction, on extrait l'halogénure de zinc avec, le cas échéant, le sel d'ammonium et/ou l'amine éventuellement contenus et la triméthylhydroquinone non convertie de la solu tion de tocophérol obtenue à l'aide d'un mélange d'eau et de méthanol à des proportions relatives en poids d'environ 4 : 1 à 1 : 10 et on recycle l'extrait ainsi obtenu, après concentration, à l'état de solution ou de bouillie pompable éventuellement chauffée dans les opérations en quantité telle que dans le mélange de réaction, après addition du complément nécessaire d'halogénure de zinc pour la réaction subséquente, il n'y ait pas plus de 3 mol d'eau par mol d'halogénure de zinc.
